# EUROPÄISCHE PATENTSCHRIFT

(11) **EP 1 294 742 B1**
(45) Veröffentlichungstag und Bekanntmachung des Hinweises auf die Patenterteilung: **31.03.2004**
(21) Anmeldenummer: 01964989.6
(22) Anmeldetag: 15.06.2001
(51) Int. Cl.: C07K 5/062, C07C 281/06, A61K 38/05, A61P 35/00, G01N 33/68

(54) **UROKINASE-HEMMSTOFFE**
UROKINASE INHIBITORS
INHIBITEURS DE L'UROKINASE

(30) Priorität: 15.06.2000 DE 10029014
(43) Veröffentlichungstag der Anmeldung: 26.03.2003
(73) Patentinhaber: Curacyte AG, 80339 München (DE)
(72) Erfinder: STÜRZEBECHER, Jörg, 99094 Erfurt (DE); STEINMETZER, Torsten, 07743 Jena (DE); KÜNZEL, Sebastian, 07743 Jena (DE); SCHWEINITZ, Andrea, 07745 Jena (DE)
(74) Vertreter: Bösl, Raphael, Dr. rer. nat., Dipl.-Chem.
(86) Internationale Anmeldenummer: PCT/EP2001/006789
(87) Internationale Veröffentlichungsnummer: WO 2001/096286

(56) Entgegenhaltungen:
- WO-A-00/05245
- DE-A- 4 243 858
- US-A- 5 863 929
- STÜRZEBECHER J ET AL.: "3-Amidinophenylalanine-based Inhibitors of Urokinase" BIOORGANIC & MEDICINAL CHEMISTRY LETTERS, Bd. 9, 1999, Seiten 3147-3152, XP002193206
- KÜNZEL S ET AL.: "4-Amidinobenzylamine-Based Inhibitors of Urokinase" BIOORGANIC & MEDICINAL CHEMISTRY LETTERS, Bd. 12, 2002, Seiten 645-648, XP002193207

## Beschreibung

Die Erfindung betrifft neue Hemmstoffe der Urokinase zur Behandlung von malignen Tumoren und der Metastasierung.

Die Ausbreitung und Metastasierung solider Tumoren in umgebendem Gewebe wird durch ihre Fähigkeit, die extrazelluläre Matrix in der Umgebung der Tumorzelle abzubauen bzw. die Basalmembran zu durchdringen, ermöglicht. In diesem Prozess kommt neben verschiedenen Matrixmetalloproteinasen und Cathepsinen vor allem dem Plasminogenaktivator Urokinase (uPA) eine zentrale Bedeutung zu (P. Mignatti und D.B. Rifkin, Physiol. Rev. 73, 161-195, 1993). So bewirkt uPA die Aktivierung von Plasminogen; das entstehende Plasmin kann die Komponenten der extrazellulären Matrix (Fibrin, Fibronektin, Laminin, Proteoglykane u.a.) abbauen sowie Metalloproteasen und pro-Urokinase zu uPA aktivieren (U. Reuning et al., Int. J. Oncol. 13, 893-906, 1998).

Sowohl pro-Urokinase als auch uPA binden an den uPA-Rezeptor (uPAR), einen an der Zelloberfläche lokalisierten, spezifischen Rezeptor. Dadurch wird eine Verstärkung und Fokussierung der Aktivität von uPA und damit der Plasminogenaktivierung in der direkten Umgebung der Tumorzelle erreicht. Sowohl in zellbiologischen Studien als auch in Tiermodellen konnte die Bedeutung dieses zellassoziierten Plasminogenaktivator-Systems für Tumorwachstum und -ausbreitung gezeigt werden. So wird das invasive Potential von Tumorzellen bei Hemmung der enzymatischen Aktivität von uPA durch die natürlichen Inhibitoren PAI-1 und PAI-2 herabgesetzt (J.-F. Cajot et al., Proc. Natl. Acad. Sci. USA 87, 6939-6943, 1990; M. Baker et al., Cancer Res. 50, 4876-4684, 1990). In Hühnerembryos konnte durch Zugabe von Antikörpern gegen uPA die durch menschliche Karzinomzellen verursachte Bildung von Lungenmetastasen fast vollständig verhindert werden (L. Ossowski et al., Cell 35, 611-619, 1983).

Die Faktoren des Plasminogenaktivator-Systems (uPA, uPAR, PA1-1 und PAI-2) sind in den letzten Jahren in Hinsicht ihrer klinischen Relevanz für die Prognose von Patienten mit soliden malignen Tumoren intensiv untersucht worden. Insbesondere der Gehalt von uPA im Gewebe verschiedener Tumoren erwies sich als ein Prognosefaktor. So haben Patienten mit hohem uPA-Spiegel eine schlechtere Prognose als solche mit niedriger uPA-Konzentration im Tumor (M. Schmitt et al., Thromb. Haemost. 78, 285-296, 1997; R.W. Stephens et al., Breast Cancer Res. Treat. 52, 99-111, 1998). Auch eine erhöhte Konzentrationen an uPAR im Tumorgewebe korreliert mit einer schlechten Prognose (H. Pedersen et al., Cancer Res. 54, 4671-4675, 1994; C. Duggan et al., Int. J. Cancer 61, 597-600, 1995).

Aus den Befunden zum prognostischen Wert des uPA- und uPAR-Gehaltes im Tumorgewebe kann angenommen werden, dass synthetische uPA-Inhibitoren in der Lage sind, Invasion und Ausbreitung von Tumorzellen zu unterdrücken. Die Zahl der bisher bekannten uPA-Hemmstoffe ist allerdings relativ klein. Die Mehrzahl besitzt nur eine geringe Spezifität und Wirkungsstärke, wie es für verschiedene Benzamidin- und β-Naphthamidin-Derivate zutrifft (J. Stürzebecher und F. Markwardt, Pharmazie 33, 599-602, 1978). Das von Vassalli und Belin (FEBS Letters 214, 187-191, 1997) als uPA-Hemmstoff beschriebene Amilorid ist zwar ein spezifischer, aber schwacher Inhibitor von uPA (Kᵢ = 7 µM).

Stärker wirksame uPA-Inhibitoren wurden mit 4-substituierten Benzothiophen-2-carboxamidinen (Kᵢ = 0,16 µM für Verbindung 623) gefunden. Hemmstoffe dieses Typs inaktivieren auch uPA, die an uPAR gebunden ist (M.J. Towle et al., Cancer Res. 53, 2553-2559, 1993). Die Benzothiophen-Derivate sind sehr spezifisch, ihre Hemmwirkung gegenüber Plasmin und dem Plasminogenaktivator vom Gewebetyp (tPA) ist gering, allerdings ist die Synthese von Verbindungen dieses Typs sehr aufwändig.

Eine vergleichbare Spezifität haben auch 4-Aminomethylphenylguanidin-Derivative, deren Hemmwirkung gegenüber uPA (Kᵢ = 2,4 µM für die wirksamste Verbindung) allerdings vergleichsweise gering ist (S. Sperl et al., Proc. Natl. Acad. Sci. USA 97, 5113-5118, 2000).

Im Gegensatz dazu erreichen Nα-Triisopropylphenylsulfonyl-3-amidinophenylalanin-Derivate mikromolare Kᵢ-Werte (0.41 µM für die wirksamste Verbindung), sind allerdings sehr unspezifische uPA-Hemmstoffe, mit gleicher oder stärkerer Wirkung werden Trypsin, Thrombin und Plasmin inhibiert (J. Stürzebecher et al., Bioorg. Med.

Letters 9, 3147-3152, 1999). Sehr wirksame uPA-Hemmstoffe sind in der WO 99/05096 mit weiterentwickelten β-Naphthamidinen offenbart. Es werden IC₅₀-Werte im nanomolaren Bereich beschrieben, allerdings keine Angaben zur Selektivität und der biologischen Wirksamkeit gemacht.

Bisher wurden nur wenige Peptide als uPA-Hemmstoffe beschrieben, die sich von der Substrat-Sequenz ableiten. Kettner und Shaw (Methods in Enzymology, 80, 826-842, 1981) beschrieben Chlormethylketone, die zwar uPA irreversibel hemmen, aber nicht für in vivo-Anwendung geeignet sind.

In der EP 18 32 71 sind Lysin-Derivate offenbart, die eine gewisse uPA-Hemmung bewirken, allerdings auch andere vergleichbare Enzyme hemmen und damit nur sehr speziell bzw. eingeschränkt für medizinische Zwecke verwendbar sind. Gleiches gilt für die in der WO 95/17 8 85 als uPA-Inhibitoren beschriebenen niedermolekularen Polypeptide (ca. 50 Aminosäuren), die sich von natürlichen Hemmstoffen ableiten. Auf Grund ihres Peptidcharakters und der Molekülgröße ist ihr in vivo-Einsatz stark eingeschränkt.

In jüngster Zeit wurden allerdings in WO 00/05 2 45 Peptidylaldehyde mitgeteilt, die C-terminal ein Argin und in P3 ein D-Serin enthielten und uPA wirksam hemmten. Jedoch bedingt die Aldehydfunktion Instabilität und ein geringe Selektivität. Nach Acylierung des Ser-Hydroxyl konnte für die Leitverbindung iBuOCO-D-Ser-Ala-Arg-H nach s.c.-Gabe eine relative Bioverfügbarkeit von 87 % beobachtet werden (S. Y. Tamura et al. Bioorg. Med. Chem. Lett. 10, 983-987, 2000).

Ferner wurden bei der Suche nach Hemmstoffen des Thrombins, einem mit uPA verwandten Enzym, mit Tripeptid-Derivaten vom D-Phe-Pro-Arg-Typ sowohl in Hinblick auf Hemmwirkung als auch Bioverfügbarkeit bemerkenswerte Fortschritte erzielt; wenn C-terminal Agmatin, trans-4-Aminomethyl-cyclohexylamin oder 4-Amidinobenzylamin eingebaut wurde. Es wurden picomolare Kᵢ-Werte erreicht und die orale Bioverfügbarkeit verbessert (T.J. Tucker et al., J. Med. Chem. 40, 1565-1569 und 3687-3693, 1997); allerdings wurden keine uPA-Hemmstoffe gefunden. So hemmt Melagatran, das C-terminal einen 4-Amidino-benzylamid-Rest besitzt, Trypsin (Kᵢ = 4,0 nM) und Thrombin (Kᵢ = 2,0 nM) sehr unspezifisch, während die Hemmung von uPA mit einem Kᵢ = 6,3 µM drei Größenordnungen schwächer ist (D. Gustafsson et al., Blood Coagul. Fibrinolysis 7, 69-79, 1996; WO 94/29336).

Der Erfindung liegt die Aufgabe zu Grunde, einen auch für therapeutische Anwendungen geeigneten Wirkstoff anzugeben, der Urokinase mit hoher Aktivität und Spezifität hemmt und der mit möglichst geringem Syntheseaufwand herstellbar ist.

Überraschend wurde gefunden, dass acyliertes Amidino-benzylamin gemäß der im Patentanspruch 1 angeführten allgemeinen Formel I Urokinase sehr wirksam und selektiv hemmt : 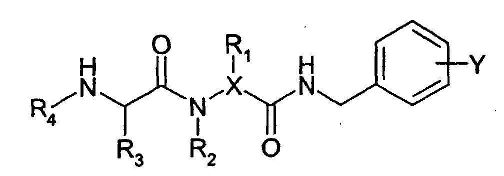 in denen der Substituent Y in 3- oder 4-Position vorkommen kann und eine Amidino-Gruppe 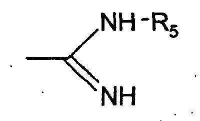 darstellt, in welcher R₅ ein H, ein OH oder einen Carbonylrest -CO-R₆ bzw. Oxycarbonylrest -COO-R₆ besitzt, wobei R₆ ein verzweigtes oder unverzweigtes Alkyl mit 1-16 C-Atomen, ein substituierter oder unsubstituierter Aryl- oder Heteroarylrest oder ein substituierter oder unsubstituierter Aralkyl- oder Heteroaralkylrest sein kann,
X eine CH-Gruppe oder N ist,
R₁ als H bzw. als ein verzweigtes oder unverzweigtes Alkyl mit 1-8 C-Atomen oder als ein (CH₂)ₙ-OH mit n=1-5 ausgebildet ist,
R₂ ein H oder ein verzweigtes oder unverzweigtes Alkyl mit 1-8 Atomen ist,
R₃ als ein (CH₂)ₙ-OH mit n=1-5 ausgebildet ist oder ein verzweigtes oder unverzweigtes Alkyl mit 1-8 C-Atomen ist bzw. als Prodrug-Form mit einem (CH₂)ₙ-OX und n=1-5 vorliegt, wobei X ein Alkyl-, Aralkyl- bzw. Aralkylcarbonyl- oder ein entsprechender Oxycarbonyl-Rest sein kann, und R₄ einen Sulfonylrest -SO₂-R₇, einen Carbonylrest -CO-R₇, einen Oxycarbonylrest -COO-R₇ oder ein H darstellt, wobei
R₇ ein verzweigtes oder unverzweigtes Alkyl mit 1-16 C-Atomen, ein substituierter oder unsubstituierter Aryl- oder Heteroarylrest, ein substituierter oder unsubstituierter Aralkyl- oder Heteroaralkylrest, ein Adamantyl- oder ein Campherrest ist.

Insbesondere fallen darunter Verbindungen des 4-Amidino-benzylamins, bei denen X, R₁, R₂ und R₃ natürliche und/oder unnatürliche Aminosäuren ergeben. Einen besonders aktiven Urokinase-Hemmstoff bildet dabei Amidino-benzylamin, wenn die Amidinogruppe in 4-Position steht und daran die Aminosäuren Gly und D-Ser sowie als R₄ ein Aryl- bzw. Aralkylsufonyl-Rest gebunden sind.

Ester, insbesondere solche mit Oxycarbonsäuren, können als Prodrug eingesetzt werden, wenn sie im Verlauf der enteralen Aufnahme hydrolysiert werden. Es wurde auch überraschend gefunden, dass einige dieser Oxycarbonyl-Derivate der erfindungsgemäßen Verbindungen ebenfalls sehr starke Urokinase-Hemmstoffe sind.

Neben Urokinase wurden durch die Glycin-Derivate andere Enzyme deutlich weniger gehemmt, so dass diese erfindungsgemäßen Derivate des Amidino-benzylamins eine neue Gruppe von hochaktiven und sehr selektiven uPA-Hemmstoffen darstellen. Im Gegensatz dazu hemmen Verbindungen, die als R₁ kein H tragen, (z. B. Alanin-Deriate) Urokinase nicht mehr selektiv, sondern sind auch starke Hemmstoffe von Trypsin, Thrombin und Plasmin.

Die Verbindungen liegen in der Regel als Salze mit Mineralsäuren, bevorzugt als Hydrochloride, vor oder als Salze mit geeigneten organischen Säuren.

Die erfindungsmässigen Verbindungen finden Verwendung zur Herstellung von oral, subkutan, intravenös bzw. transdermal verabreichbaren Arzneimitteln für die Tumorbekämpfung, wobei die erfindungsmässigen Urokinase-Hemmstoffe als Arzneimittel in Form von Tabletten, Dragees, Kapseln, Pellets, Suppositorien, Lösungen oder Pflaster eingesetzt werden.

Ferner können die erfindungsmässigen Urokinase-Hemmstoffe zur Herstellung eines Mittels zur Diagnose bei Tumoruntersuchungen verwendet werden.

Die Verbindungen der allgemeinen Formel I können, wie nachfolgend beschrieben, nach bekannten Verfahren auf relativ einfache Weise hergestellt werden:

Die Ausgangsverbindung 4-Cyanobenzylamin wird über Gabrielsynthese (G. Wagner und I. Wunderlich, Pharmazie 32, 76-77, 1977; B.C. Bookser und T.C. Bruice, J. Am. Chem. Soc. 113, 4208-4218, 1991) aus 4-Cyanobenzylbromid hergestellt. Aus dem so hergestellten 4-Cyanobenzylamin wird das Boc-geschützte Acetyloxamidino-benzylamin gewonnen. Die Ankopplung der weiteren Aminosäuren und der Schutzgruppe R₄ erfolgt mittels Standardkopplungsmethoden mit Boc als N-terminale Schutzgruppe. Die zweite Aminosäure kann auch direkt als N-aryl- bzw. N-aralkyl-sulfonyl-geschützte Aminosäure gekoppelt werden. Die Peptidanaloga werden sequentiell, beginnend vom Acetyloxamidino-benzylamin, aufgebaut. Zur Synthese der entsprechenden Ester wird die Zielverbindung mit derm entsprechenden Säurechlorid umgesetzt. Die meisten Produkte kristallisierten gut und lassen sich damit einfach reinigen. Die Reinigung der Hemmstoffe erfolgt in der letzten Stufe über präparative, reversed-phase HPLC.

Die Erfindung soll nachstehend anhand von zwei Ausführungsbeispielen näher erläutert werden:

### Ausführungsbeispiel 1:

### Synthese von Benzylsulfonyl-D-Ser-Gly-4-Amidino-benzylamid x HCl

### 1.1 Boc-4-Cyano-benzylamid

20 g (0,151 mol) 4-Cyano-benzylamin wurden in 300 ml H₂O, 150 ml Dioxan und 150 ml 1 N NaOH gelöst. Unter Eiskühlung wurden 37,5 ml Di-tert.-butyl-dicarbonat zugetropft und eine Stunde bei 0 °C sowie weitere 24 Std. bei Raumtemperatur gerührt. Das Dioxan wurde im i.V. entfernt und der wässrige Rückstand 3-mal mit Essigester extrahiert. Die vereinigten Extrakte wurden 3-mal mit 5 %-iger KHSO₄- und 3-mal mit gesättigter NaCl-Lösung gewaschen, über Na₂SO₄ getrocknet und i.V. eingeengt (weiße Kristalle). HPLC: Acetonitril/H₂O, Elution bei 44,1 % Acetonitril; Ausbeute: 30,48 g (0,131 mol), 87 %.

### 1.2 Boc-4-Acetyloxamidino-benzylamid

Nach Judkins et al. (Synthetic Comm. 26, 4351-4367, 1996) wurden 30,48 g (0,131 mol) Boc-4-Cyano-benzylamid mit 13,65 g (0,197 mol) Hydroxylamin x HCl und 34 ml (0,197 mol) DIEA in 300 ml abs. Ethanol gelöst. Es wurde 2 Std. unter Rückfluss gekocht und über Nacht bei Raumtemperatur gerührt. Danach wurde der Ansatz i.V. eingeengt, der Rückstand in ca. 200 ml Essigsäure gelöst und mit 18,67 ml (0,197 mol) Essigsäureanhydrid versetzt. Nach 1 Std. wurde erneut eingeengt, in Essigester gelöst und bei 0 °C je 3-mal mit 5 %iger KHSO₄- und gesättigter NaCl-Lösung gewaschen. Nach dem Trocknen über Na₂SO₄ und Einengen i.V. fiel ein weißes Pulver an. HPLC: Acetonitril/H₂O, Elution bei 32,0 % Acetonitril; Ausbeute: 31,3 g (0,102 mol) 78 %.

### 1.3 4-Acetyloxamidino-benzylamin x HCl

5 mmol Boc-4-Acetyloxamidino-benzylamid werden in 20 ml 1 N HCl in Eisessig gelöst und 45 min bei Raumtemperatur stehen gelassen. Dann wird i.V. weitgehend eingeengt, das Produkt mit trockenem Diethylether gefällt, abgefrittet, nochmals mit frischem Ether gewaschen und i.V. getrocknet. Auf Grund der quantitativen Umsetzung wurde das Produkt ohne weitere Reinigung für den nächsten Syntheseschritt eingesetzt.

### 1.4 Boc-Gly-4-Acetyloxamidino-benzylamid

Die Kopplung von Boc-Gly-OH (Orpegen, Heidelberg) an 4-Acetyloxamidino-benzylamin erfolgte nach Frérot et al. (Tetrahedron 47, 259 ff., 1991). Dazu wurden 2,064 g (9,3 mmol) 4-Acetyloxamidino-benzylamin x HCl und 1,629 g (9,3 mmol) Boc-Gly-OH in ca. 25 ml DMF gelöst. Bei 0 °C wurden dann 4,84 g (9,3 mmol) PyBOP und 3,878 ml (27,9 mmol) TEA zugegeben und der pH-Wert mit TEA auf 9 eingestellt. Nach 1 Std. Rühren bei Raumtemperatur wurde i.V. eingeengt, in Essigester aufgenommen und je 3-mal sauer, basisch und neutral gewaschen, getrocknet und eingeengt. Ausbeute: 3 g (8,2 mmol) 88%.

### 1.5 Boc-Gly-4-Amidino-benzylamid x AcOH

3 g (8,2 mmol) Boc-Gly-4-Acetyloxamidino-benzylamid wurden in 200 ml 90 %iger Essigsäure gelöst. Anschließend wurden unter Argon 300 mg 10 % Palladium auf Aktivkohle zugesetzt. Argon wurde durch eine Wasserstoffatmosphäre ersetzt und der Ansatz unter kräftigem Rühren 24 Std. hydriert. Der Katalysator wurde abfiltriert und das Filtrat i.V. eingeengt. Ausbeute: 2,9 g (7,9 mmol) 96 %.

### 1.6 H-Gly-4-Amidino-benzylamid x 2 HCl

2,9 g (7,9 mmol) Boc-Gly-4-Amidino-benzylamid wurden in 100 ml 1 N HCl in Eisessig gelöst und 45 min bei Raumtemperatur stehen gelassen. Dann wurde i.V. weitgehend eingeengt und mit trockenem Diethylether gefällt, danach abgefrittet und das Produkt nochmals mit frischem Ether gewaschen. Nach Trocknen des Produkts i.V. wurde es ohne weitere Aufreinigung für die Synthese nach Punkt 1.8 eingesetzt.

### 1.7 Benzylsulfonyl-D-Ser(Bz)-OH

229 mg (1,173 mmol) H-D-Ser(Bz)-OH (Bachem, Heidelberg) und 408 µl (2,345 mmol) DIEA wurden in 50 ml 50 % Acetonitril gelöst. Dann wurden 335 mg (1,76 mmol) Benzylsulfonylchlorid zugegeben und 12 Std. bei Raumtemperatur gerührt. Es wurde i.V. eingeengt, mit Essigester aufgenommen und je 3-mal sauer und neutral gewaschen. Nach Trocknen über Natriumsulfat wurde i.V. eingeengt. Ausbeute: 289 mg (0,827 mmol) 71 %.

### 1.8 Benzylsulfonyl-D-Ser(Bz)-Gly-4-Amidino-benzylamid x TFA

151 mg (0,433 mmol) Benzylsulfonyl-D-Ser(Bz)-OH und 121 mg (0,433 mmol) H-Gly-4-Amidino-benzylamid x 2 HCl wurden in wenig abs. DMF gelöst. Unter Eiskühlung wurden 225 mg (0,433 mmol) PyBOP und 230 µl (1,32 mmol) DIEA zugegeben. Nach 1 Std. Rühren bei Raumtemperatur wurde i.V. eingeengt und das Produkt über HPLC gereinigt (Acetonitril/H₂O, 0,1 % Trifluoressigsäure, Elution bei 37,4 % Acetonitril).
Ausbeute: 232 mg (0,356 mmol) 82 %.

### 1.9 Benzylsulfonyl-D-Ser-Gly-4-Amidino-benzylamid x HCl

50 mg HPLC-gereinigtes Benzylsulfonyl-D-Ser(Bz)-Gly-4-Acetyloxamidino-benzylamid x TFA werden in 50 ml 90 %iger Essigsäure gelöst und mit 50 mg 10 % Palladium auf Aktivkohle 48 Std. bei Raumtemperatur hydriert. Danach wird der Katalysator abfiltriert und das Filtrat i.V. eingeengt. Das Produkt wird über HPLC gereinigt (Acetonitril/H₂O mit 0,1 % TFA, Elution auf analytischer HPLC bei 21,4 % Acetonitril) und mittels Ionenaustauscher in die HCl-Form überführt.
Ausbeute: 20 mg (0,041 mmol) 54 %.

### 1.10 Benzylsulfonyl-D-Ser(COO-Isobutyl)-Gly-4-Amidino-benzylamid x HCl

30 mg (0,062 mmol) Benzylsulfonyl-D-Ser-Gly-4-Amidino-benzylamid x HCl werden in 3 ml Pyridin unter Zusatz von 1 ml Acetonitril bei Raumtemperatur gelöst. Unter Eiskühlung werden 16,1 µl (0,124 mmol) Chlorameisensäureisobutylester hinzugegeben. Der Ansatz wird 30 Minuten unter Eiskühlung und anschließend über Nacht bei Raumtemperatur gerührt. Das Lösungsmittel wird i.V. entfernt und das Produkt über HPLC gereinigt (Elution auf analytischer HPLC bei 37,9 % Acetonitril) und mittels Ionenaustauscher in die HCl-Form überführt.
Ausbeute: 13 mg (0,022 mmol) = 36 %.

### Ausführungsbeispiel 2:

| Hemmung von Urokinase durch ausgewählte Verbindungen mit Y = Amidino | | | | | | |
|---|---|---|---|---|---|---|
| R₄ | Konfiguration R₃ | R₃ | R₂ | X-R₁ | Position Amidino | Kᵢ, µM |
| H | L | CH₂-OH | H | CH₂ | 4 | 21 |
| Boc | L | CH₂-OH | H | CH₂ | 4 | 23 |
| H | D | CH₂-OH | H | CH₂ | 4 | 12 |
| Ac | D | CH₂-OH | H | CH₂ | 4 | 41 |
| Bz-SO₂ | D | CH₂-OH | H | CH₂ | 4 | 0,036 |
| CMe-SO₂ | D | CH₂-OH | H | CH₂ | 4 | 0,048 |
| Bz-SO₂ | D | CH₂-O-Bz | H | CH₂ | 4 | 0,84 |
| Bz-SO₂ | D | CH₂-OH | H | CH-CH₃ | 4 | 0,0077 |
| Bz-SO₂ | D | CH₂-O-COO-CH₃ | H | CH₂ | 4 | 0,39 |
| Bz-SO₂ | D | CH₂-O-COO-iBu | H | CH₂ | 4 | 0,50 |
| Bz-SO₂ | D | CH₂-O-COO-iBu | H | CH -CH₃ | 4 | 0,043 |
| H | D | CH₂-O-Bz | H | CH₂ | 3 | > 1000 |
| Boc | D | CH₂-O-Bz | H | CH₂ | 3 | > 1000 |
| Bz-SO₂ | D | CH₂-O-Bz | H | CH₂ | 3 | > 1000 |

### Bestimmung der Hemmwirkung:

Zur Bestimmung der Hemmwirkung wurden 200 µI Tris-Puffer (0,05 M, 0,154 M NaCI, 5 % Ethanol, pH 8,0; enthält den Inhibitor), 25 µI Substrat (Bz-βAla-Gly-Arg-pNA in H₂O) und 50 µl sc-Urokinase bei 25 °C inkubiert. Nach 3 min wurde die Reaktion durch Zugabe von 25 µI Essigsäure (50%) unterbrochen und die Absorption bei 405 nm mittels Microplate Reader (Dynatech MR 5000) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen.

### Verwendete Abkürzungen:

- Ac: Acetyl
- Boc: tert.-Butyloxycarbonyl
- Bz: Benzyl
- DIEA: Diisopropylethylamin
- DMF: N,N-Dimethylformamid
- i.V.: im Vakuum
- PyBOP: Benzotriazol-1-yl-N-oxy-tris(pyrrolidino)phosphoniumhexafluorophosphat
- TEA: Triethylamin
- TFA: Trifluoressigsäure
- THF: Tetrahydrofuran
- CMe: Cyclohexylmethyl
- iBu: iso-butyl

## Patentansprüche

1. Urokinase-Hemmstoffe der allgemeinen Formel I: 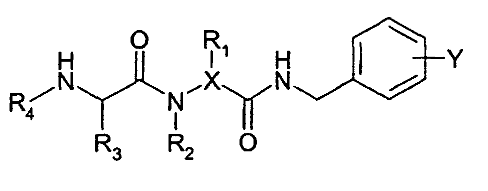 in denen der Substituent Y in 3- oder 4-Position vorkommen kann und eine Amidino-Gruppe 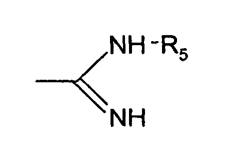 darstellt, in welcher R₅ ein H, ein OH oder einen Carbonylrest -CO-R₆ bzw. Oxycarbonylrest -COO-R₆ besitzt, wobei R₆ ein verzweigtes oder unverzweigtes Alkyl mit 1-16 C-Atomen, ein substituierter oder unsubstituierter Aryl- oder Heteroarylrest oder ein substituierter oder unsubstituierter Aralkyl- oder Heteroaralkylrest sein kann,
X eine CH-Gruppe oder N ist,
R₁ als H bzw. als ein verzweigtes oder unverzweigtes Alkyl mit 1-8 C-Atomen oder als ein (CH₂)ₙ-OH mit n=1-5 ausgebildet ist,
R₂ ein H oder ein verzweigtes oder unverzweigtes Alkyl mit 1-8 Atomen ist,
R₃ als ein (CH₂)ₙ-OH mit n=1-5 ausgebildet ist oder ein verzweigtes oder unverzweigtes Alkyl mit 1-8 C-Atomen ist bzw. als Prodrug-Form mit einem (CH₂)ₙ-OX und n=1-5 vorliegt, wobei X ein Alkyl-, Aralkyl- bzw. Aralkylcarbonyl- oder ein entsprechender Oxycarbonyl-Rest sein kann,
und R₄ einen Sulfonylrest -SO₂-R₇, einen Carbonylrest -CO-R₇, einen Oxycarbonylrest -COO-R₇ oder ein H darstellt, wobei
R₇ ein verzweigtes oder unverzweigtes Alkyl mit 1-16 C-Atomen, ein substituierter oder unsubstituierter Aryl- oder Heteroarylrest, ein substituierter oder unsubstituierter Aralkyl- oder Heteroaralkylrest, ein Adamantyl- oder ein Campherrest ist.

2. Urokinase-Hemmstoffe gemäß Anspruch 1, **dadurch gekennzeichnet, dass** im Amidino-benzylamid die Amidinogruppe in 4-Position steht und dass daran die Aminosäuren Gly und D-Ser sowie als R₄ ein Aryl- oder ein Aralkylsulfonyl-Rest gebunden sind.

3. Verwendung der Urokinase-Hemmstoffe gemäß Anspruch 1 oder 2 zur Herstellung von oral, subkutan, intravenös bzw. transdermal verabreichbaren Arzneimitteln für die Tumorbekämpfung.

4. Verwendung gemäß Anspruch 3, **dadurch gekennzeichnet, dass** die Urokinase-Hemmstoffe als Arzneimittel in Form von Tabletten, Dragées, Kapseln, Pellets, Suppositorien, Lösungen oder Pflaster eingesetzt werden.

5. Verwendung der Urokinase-Hemmstoffe gemäß Anspruch 1 zur Herstellung eines Mittels zur Diagnose bei Tumoruntersuchungen.

## Claims

1. Urokinase inhibitors of the formula I: 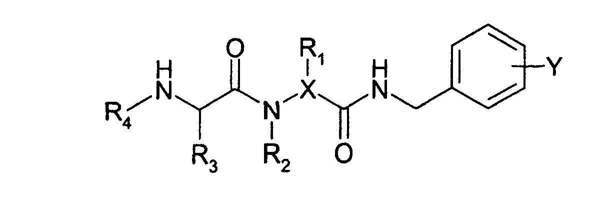 in which the substituent Y can be present in the 3 position or 4 position and is an amidino group 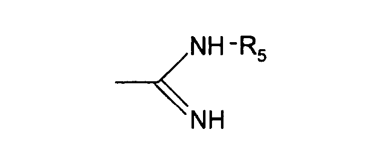 in which R₅ is an H, an OH or a carbonyl radical -CO-R₆ or an oxycarbonyl radical -COO-R₆, where R₆ can be a branched or unbranched alkyl having 1-16 C atoms, a substituted or unsubstituted aryl or heteroaryl radical or a substituted or unsubstituted aralkyl or heteroaralkyl radical,
X is a CH group or N,
R₁ is H or a branched or unbranched alkyl having 1-8 C atoms or a (CH₂)ₙ-OH in which n = 1-5,
R₂ is an H or a branched or unbranched alkyl having 1-8 C atoms,
R₃ is a (CH₂)ₙ-OH in which n = 1-5 or is a branched or unbranched alkyl having 1-8 C atoms or is present in prodrug form and is a (CH₂)ₙ-OX in which n = 1-5, where X can be an alkyl, aralkyl or aralkylcarbonyl radical or a corresponding oxycarbonyl radical,
and R₄ is a sulfonyl radical -SO₂-R₇, a carbonyl radical -CO-R₇, an oxycarbonyl radical -COO-R₇ or an H, where
R₇ is a branched or unbranched alkyl having 1-16 C atoms, a substituted or unsubstituted aryl or heteroaryl radical, a substituted or unsubstituted aralkyl or heteroaralkyl radical or an adamantyl radical or a camphor radical.

2. Urokinase inhibitors as claimed in claim 1, **characterized in that** the amidino group in the amidinobenzylamide is in the 4 position and **in that** the amino acids Gly and D-Ser and also an arylsulfonyl radical or an aralkylsulfonyl radical, as R₄, are bonded to it.

3. The use of the urokinase inhibitors as claimed in claim 1 or 2 for producing pharmaceuticals, which can be administered orally, subcutaneously, intravenously or transdermally, for controlling tumors.

4. The use as claimed in claim 3, **characterized in that** the urokinase inhibitors are employed as pharmaceuticals in the form of tablets, sugar-coated tablets, capsules, pellets, suppositories, solutions or plasters.

5. The use of a urokinase inhibitor as claimed in claim 1 as a diagnostic agent for investigating tumors.

## Revendications

1. Des inhibiteurs d'urokinase de formule générale I : 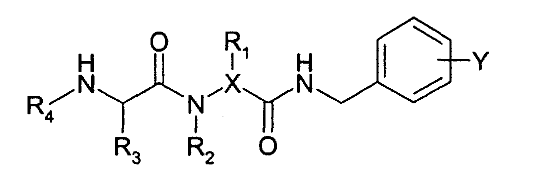 dans lesquels le substituant Y peut se trouver en position 3 ou en position 4 et représente un group amidino 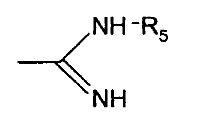 dans lequel R₅ représente un atome d'hydrogène, un groupe OH ou un reste carbonyle -CO-R₆ ou oxycarbonyle -COO-R₆, où R₆ est un radical alkyle linéaire ou ramifié comportant 1 à 16 atomes de carbone, un reste aryle ou hétéroaryle substitué ou non substitué ou bien un reste arylalkyle ou hétéroarylalkyle substitué ou non substitué,
X est un groupe CH ou un N,
R₁ est un atome d'hydrogène ou un radical alkyle linéaire ou ramifié comportant 1 à 8 atomes de carbone ou un groupe (CH₂)ₙ-OH où n=1 à 5,
R₂ est un atome d'hydrogène ou un radical alkyle linéaire ou ramifié comportant 1 à 8 atomes de carbone,
R₃ est un groupe (CH₂)ₙ-OX et n=1 à 5, où X est un reste alkyle, arylalkyle ou arylalkylcarbonyle ou un reste oxycarbonyle correspondant,
et R₄ est un reste sulfonyle-SO₂-R₇, un reste carbonyle-CO-R₇, un reste oxycarbonyle -COO-R₇ ou un atome d'hydrogène, où
R₇ est un alkyle linéaire ou ramifié comportant 1 à 16 atomes de carbone, un reste aryle ou hétéroaryle substitué ou non substitué, un reste arylaklyle ou hétéroarylalkyle substitué ou non substitué, un reste adamantyle ou camphre.

2. Des inhibiteurs d'urokinase selon la revendication 1, **caractérisé en ce que**, dans l'amidino-benzylamide, le groupe amidino est en position 4 et que y sont fixés les acides aminés Gly et D-Ser et que R₄ est un reste arylsulfonyle ou arylalkylsulfonyle.

3. Utilisation d'un inhibiteur d'urokinase selon l'une quelconque des revendications 1 ou 2 pour la préparation d'un médicament utilisable par voie orale, sous-cutanée, intraveineuse ou transdermique pour la lutte contre les tumeurs.

4. Utilisation selon la revendication 3, **caractérisée en ce que** l'inhibiteur d'urokinase est utilisé comme médicament sous forme de comprimés, dragées, capsules, granulés, suppositoires, solutions ou emplâtres.

5. Utilisation de l'inhibiteur d'urokinase selon la revendication 1, dans la préparation de moyen de diagnostic de recherches tumorales.
